(19) Europäisches Patentamt
European Patent Office
Office européen des brevets

(11) **EP 1 933 129 B1**

(12) **EUROPÄISCHE PATENTSCHRIFT**

(45) Veröffentlichungstag und Bekanntmachung des Hinweises auf die Patenterteilung:
**20.02.2013 Patentblatt 2013/08**

(51) Int Cl.:
***G01N 15/10*** *(2006.01)*      ***G01N 33/28*** *(2006.01)*

(21) Anmeldenummer: **07024192.2**

(22) Anmeldetag: **13.12.2007**

(54) **Verfahren zum Erfassen von Partikeln in einer strömenden Flüssigkeit**

Method for measuring particles in a fluid stream

Procédé destinés à détecter des particules dans un liquide s'écoulant

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **15.12.2006 DE 102006059437**
**21.08.2007 DE 102007039435**

(43) Veröffentlichungstag der Anmeldung:
**18.06.2008 Patentblatt 2008/25**

(73) Patentinhaber: **Prüftechnik Dieter Busch AG**
**85737 Ismaning (DE)**

(72) Erfinder:
• **Becker, Edwin, Dr.**
**48734 Reken (DE)**
• **Knöll, Thomas, Dr.**
**89231 Neu-Ulm (DE)**
• **Hölzl, Roland**
**81369 München (DE)**

(56) Entgegenhaltungen:
**EP-A1- 0 773 440      EP-A1- 1 979 733**
**EP-A2- 0 124 042      EP-A2- 1 189 058**
**GB-A- 2 165 650        US-A- 5 001 424**
**US-B1- 7 148 678**

EP 1 933 129 B1

## Beschreibung

Die Erfindung betrifft ein Verfahren zum Erfassen von elektrisch leitenden Partikeln in einer in einem Rohr strömenden Flüssigkeit mittels Wirbelströmen

wobei der abgeschätzte radiale Abstand der erfassten Partikel (20) von der Empfängerspulenwand bei der Abschätzung der Strömungsgeschwindigkeit der Partikel verwendet wird,

oder wobei der abgeschätzte radiale Abstand jedes erfassten Partikel (20) von der Empfängerspulenwand bei der Abschätzung der Größe der Partikel verwendet wird.

In der DE 2 108 717 A1 ist ein solches Verfahren beschrieben, wobei zwei Induktionsspulen in zwei Zweigen einer Wechselstrombrückenschaltung angeordnet sind, deren andere beiden Zweige durch die Hälften einer weiteren Spule gebildet werden. Die Spulen werden in axialer Richtung von der Flüssigkeit durchströmt und können in Strömungsrichtung hintereinander angeordnet sein, wobei die durch den Durchgang der Partikel hervorgerufenen Impedanzänderungen, bzw. der Unterschied der Impedanzänderung in beiden Spulen, ausgewertet wird. Es ist eine Anordnung gezeigt, bei welcher die Flüssigkeitsströmung in zwei parallel Teilstrecken aufgeteilt wird, die jeweils durch eine der beiden Spulen strömen, wobei dann ein axialer Versatz der Spulen nicht erforderlich ist.

Eine ähnliche Vorrichtung ist in der DE 28 40 358 A1 beschrieben.

Von der Firma momac GmbH & Co. KG, 47408 Moers, Deutschland, wird unter der Bezeichnung "metalscan" ein Gerät vertrieben, bei dem drei Spulen in Strömungsrichtung hintereinander angeordnet sind, wobei die erste und die letzte Spule als Senderspulen und die mittlere Spule als Empfängerspule wirken, um den Durchgang von elektrisch leitenden Partikeln aus einem Schmiermittelkreislauf zu erfassen. Die erste und die letzte Spule sind umgekehrt gepolt.

Weitere Vorrichtungen, bei denen das Signal von von der Flüssigkeit durchströmten Induktionsspulen zur Partikelerfassung verwendet wird, sind beispielsweise in der WO 2004/081608, WO 2004/104561, EP 0 778 937 A2 und EP 0 451 209 B1 beschrieben.

Aus der DE 39 31 497 A1 ist ein Verfahren zum induktiven Erfassen von Partikeln in Schmierstoffen bekannt, wobei eine in einer axial durchströmten Spule eingebettete Ankoppelspule resonant angeregt wird und der Durchgang von Partikeln anhand der dem Spulensystem durch die Wirbelströme entzogenen Energie erfasst wird. Dabei wird aus der Signalamplitude die Partikelgrösse bestimmt. Um eine Verfälschung der Messung durch bei Teilchendurchgang in der Spulenmitte im Vergleich zum Spulenrand abnehmende Empfindlichkeit der Spule zu vermeiden, wird durch einen Drallerzeuger dafür gesorgt, dass die Partikel die Spule immer nahe der Spulenwand passieren.

Aus der DE 31 17 319 A1 ist die Erfassung der Fließgeschwindigkeit von flüssigem Metall mittels Wirbelstrommessung unter Verwendung ein Kreuzkorrelationsfunktion beschrieben.

In der DE 40 14 756 A1 ist die Bestimmung der Geschwindigkeit eines Körpers oder Materials mittels Wirbelstrommessung beschrieben, wobei eine Korrelationsfunktion gebildet wird.

In der US 3,575,050 und der DE 28 50 246 A1 wird erwähnt, dass es Strömungsmessgeräte auf Wirbelstrombasis gibt.

Die EP 1 979 733 A1 beschreibt eine Verfahren gemäß des Oberbegriffes von Anspruch 1. Die Druckschrift gehört zum Stand der Technik gemäß Artikel 54(3) EPÜ. Dabei wird ein Verfahren beschrieben in dem mittels zweier Sensorspulen der Durchgang von ferromagnetischen und elektrisch leitenden Partikeln detektiert wird. Diese Partikel wurden zuvor mithilfe einer Sensorspule erregt.

Die US 5 001 424 A1 beschreibt ein weiteres Verfahren zum Erfassen von magnetischen Partikeln in einem Schmiermittelkreislauf gemäß des Oberbegriffes von Anspruch 1.

Ferner ist es bekannt, bei der Wirbelstromprüfung von metallischen Werkstücken eine Spulenanordnung zu verwenden, bei welcher bei in Längsrichtung des Werkstücks beabstandete und subtraktiv geschaltete Empfängerspulen vorgesehen sind, die außen von einer koaxial dazu angeordneten Senderspule umgeben sind. Bei der Wirbelstromprüfung wird dann das Werkstück durch das Innere der beiden Empfängerspulen durchgeschoben. Die Senderspule bildet dabei die Primärseite und die Empfängerspulen die Sekundärseite einer transformatorischen Anordnung. Ein Beispiel für eine solche Anordnung ist in der EP 1 189 058 A2 zu finden.

Bei der Wirbelstromprüfung von Werkstücken wird die Tatsache ausgenutzt, dass Materialfehler in dem Werkstück die Ausbreitung von Wirbelströmen, welche mittels der Senderspule induziert werden, behindern, was sich auf das von den Wirbelströmen erzeugte elektromagnetische Feld auswirkt, das wiederum von einem Sensor erfaßt wird, bei dem es sich um die Senderspule selbst oder um mindestens eine separate Empfängerspule handeln kann. Falls nur eine einzige separate Empfängerspule vorgesehen ist, wird diese Anordnung "Absolutspule" genannt. Zwei oder mehr Meßspulen können subtraktiv geschaltet werden, was dann als "Differenzspule" bezeichnet wird und das Ausschalten beispielsweise einer Temperaturdrift ermöglicht. Falls mehr als zwei Empfängerspulen verwendet werden, wird eine solche Anordnung auch als "Multi-Differenzspule" bezeichnet.

In ähnlicher Weise verursachen elektrisch leitende Partikel in einer Flüssigkeit, welche die Spulen durchströmt, Wirbelstromverluste, die sich wiederum in einer meßbaren Impedanzänderung der Spulen niederschlagen. Auf diese Weise können mittels einer induktiven Spulenanordnung elektrisch leitende Partikel in einer in einem Rohr strömenden Flüssigkeit erfaßt werden. Dies ist besonders vorteilhaft, wenn man die Konzentration metallischer Partikel in einem Schmiermittelkreislauf einer Maschine erfassen will, um auf den Maschinenzustand rückzuschließen (die Konzentration der metallischen Partikel ist in der Regel ein Maß für den Verschleiß der Maschine).

Es ist Aufgabe der vorliegenden Erfindung, ein Verfahren zum Erfassen von elektrisch leitenden Partikeln in einer in einem Rohrstück strömenden Flüssigkeit zu schaffen, wobei eine möglichst gute Genauigkeit erzielt werden soll.

Diese Aufgabe wird erfindungsgemäß gelöst durch ein Verfahren gemäß Anspruch 1.

Bei dieser Lösung ist vorteilhaft, dass dadurch, dass die Senderspule und die Empfängerspulen eine transformatorische Anordnung bilden und die Senderspule im Bereich der Empfängerspulen angeordnet ist, alle Spulen nahe beieinander angeordnet sind und damit im wesentlichen den gleichen Umwelteinflüssen, z.B. hinsichtlich Temperatur ausgesetzt sind, was die Meßgenauigkeit erhöht.

Bevorzugte Ausgestaltungen der Erfindung sind in den Unteransprüchen angegeben.

Im folgenden wird die Erfindung beispielhaft anhand der beigefügten Zeichnungen näher erläutert. Dabei zeigen:

Fig. 1    in schematischer Weise einen Längsschnitt durch ein von einer Flüssigkeit durchströmtes Rohr, welches mit den Spulen einer Vorrichtung zum Erfassen von leitenden Partikeln in der Flüssigkeit versehen ist;

Fig. 2    ein Blockschaltbild einer solchen Vorrichtung;

Fig. 3    einen idealisierten Verlauf des Betrags des Signals der Empfängerspulen von Fig. 1, wobei die Messwerte unterhalb eines Schwellwerts abgeschnitten wurden;

Fig.4    eine schematische Darstellung der theoretischen radialen Geschwindigkeitsverteilung in einer laminaren Strömung in einem Rohr;

Fig. 5    ein Beispiel für die Abhängigkeit der Wirkbreite der Empfängerspulen von Fig. 1 von der radialen Position eines erfassten Partikels; und

Fig. 6    ein Beispiel für die Abhängigkeit der Dämpfung des Signals der Empfängerspulen von Fig. 1 von der radialen Position eines erfassten Partikels.

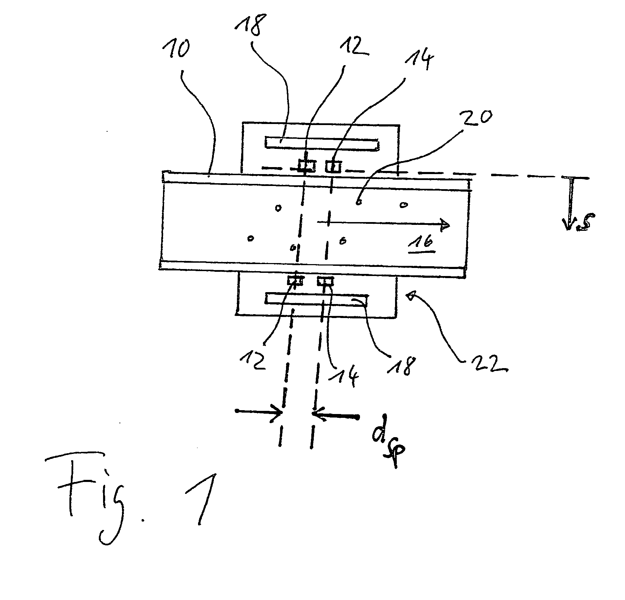

Gemäß Fig. 1 ist ein Rohrstück 10 von einer ersten induktiven Empfängerspule 12 und einer davon in axialer Richtung beabstandet angeordneten zweiten induktiven Empfängerspule 14 umgeben, so dass eine in dem Rohrstück 10 strömende Flüssigkeit 16 die Spulen 12 und 14 in axialer Richtung durchströmt. Der axiale Abstand der beiden Spulen 12, 14 und die axiale Abmessung der Spulen 12, 14 kann beispeilsweise 2 mm betragen. Die beiden Empfängerspulen 12, 14 sind außen von einer Senderspule 18 umgeben, die koaxial zu den beiden Spulen 12, 14 angeordnet ist und einen größeren Durchmesser als diese aufweist. Die axiale Abmessung der Senderspule 18 ist dergestalt, dass die beiden Empfängerspulen 12, 14 vollständig innerhalb der Senderspule 18 angeordnet sind. Vorzugsweise ist die Ausdehnung der Senderspule 18 in axialer Richtung mindestens doppelt so groß ist wie die axiale Ausdehnung der Anordnung der Empfängerspulen 12, 14, d.h. Abstand plus axiale Ausdehung der Spulen 12, 14. Die Spulen 12, 14, 18 sind in einem das Rohrstück 10 umschließenden Gehäuse 22 angeordnet.

Typischerweise ist das Rohrstück 10 Teil eines Schmiermittelkreislaufs einer Maschine, wobei es sich bei der Flüssigkeit 16 dann um ein Schmiermittel handelt, in welchem metallische Partikel vorhanden sind, bei denen es sich typischerweise um Abrieb aus bewegten Teilen der Maschine handelt. Ein typischer Wert für den Schmiermitteldurchsatz im Hauptstrom ist 10 Liter/min. Bei wesentlich höheren Durchsätzen ist es zweckmäßig, nicht im Hauptstrom, sondern in einem Nebenstrom zu messen.

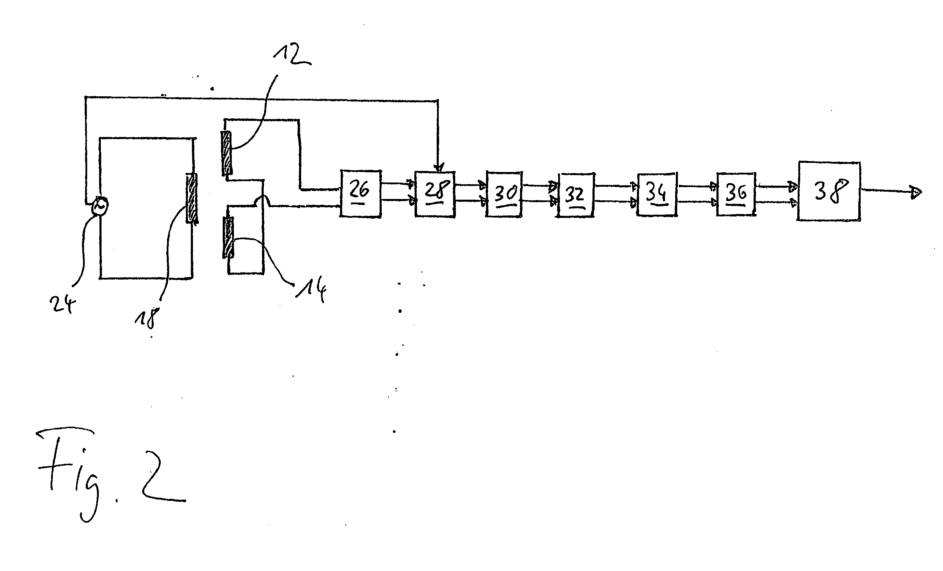

Gemäß Fig. 2 sind die beiden Empfängerspulen 12, 14 subtraktiv als Differenzspule geschaltet, d.h. sind sind gegenläufig angeordnet, so dass in den beiden Spulen 12, 14 eine Spannung mit gleichem Betrag aber entgegengesetzten Vorzeichen induziert wird.. Insgesamt bilden die Senderspule 18 und die Empfängerspulen 12, 14 eine transformatorische Anordnung, wobei die Senderspule 18 die Primärseite bildet und die Empfängerspulen 12, 14 die Sekundärseite bilden. Der Transformatorkern wird bei einer solchen Anordnung von den die Spulen 12, 14, 18 durchsetzenden Materialien bzw. Medien, d.h. Luft, dem Gehäuse 22, dem Rohr 10, sowie der Flüssigkeit 16 mit den Partikeln 20, gebildet.

Der durch die Partikel 20 verursachte Impedanzunterschied der Spulen 12, 14, d.h. der durch die momentane Anwesenheit eines Partikels 20 in einer der beiden Spulen 12, 14 (die Partikel 20 sind wesentlich kleiner als der Abstand der Spulen 12, 14) verursachte Unterschied der Impedanz der beiden Spulen 12, 14 wird durch das von den Spulen 12 und 14 ausgegebene Meßsignal abgebildet.

Die Senderspule 18 wird mittels eines Oszillators 24 mit einer geeigneten Wechselspannung beaufschlagt, die vorzugsweise im Bereich zwischen 20 und 500 kHz liegt, um über in den elektrisch leitenden Partikeln 20 induzierten Wirbelströmen ein Meßsignal der Empfängerspulen 12, 14 zu erzeugen. Das an den Spulen, 12, 14 abgegebene Meßsignal wird über eine Eingangsstufe 26 mit einem Vorverstärker einer Einheit 28 zugeführt, in welcher eine Demodulation bezüglich der Senderfrequenz des Oszillators 24 erfolgt, wobei entweder eine Betragsbildung erfolgen kann oder alternativ eine zweikanalige Demodulation mit einer Phasenverschiebung von 90° zwischen den beiden Kanälen stattfindet. In letzterem Fall ist der nachfolgende Signalpfad dann zweikanalig ausgebildet. Diese Variante ist in Fig. 2 dargestellt. Das demodulierte Signal wird dann durch ein Filter 30 geleitet, welches mittels eines Tiefpasses die Trägerfrequenz und mittels eines Hochpasses die Spulenfehlspannung ausgefiltert (grundsätzlich ergibt sich bei einer Differenzspule als Folge der Differenzbildung (die einzelnen Spulen der Differenzspule sind in der Praxis nie baugleich) die sog. Spulenfehlspannung, die das eigentliche Differenzsignal z.B. um bis zu drei Größenordnungen übersteigen kann). Das so gefilterte Signal wird dann mittels eines Verstärkers 32 verstärkt und durch einen vorzugsweise variablen Bandpaßfilter 34 geleitet, welches eventuell dem Signal überlagerte Störungen ausfiltert.

Das bandpaßgefilterte Signal durchläuft einen Phasensteller 36, der es erlaubt, die Phasenlage des Signals in einer für die Auswertung günstigen Weise einzustellen, bevor das Signal in eine Auswertungseinheit 38 eingespeist wird, welche in an sich bekannter Weise die Amplitude und die Phasenlage des von den Partikeln 20 herrührenden Meßsignals ermittelt. Dieses Signal kann beispielsweise in einer Orbitaldarstellung auf einem Bildschirm dargestellt werden. Zweckmäßigerweise ist die Auswertungseinheit 38 so ausgebildet, dass eine Zählung der erfaßten Partikeldurchgänge erfolgt, um auf die Partikelkonzentration in der Flüssigkeit 16, und damit gegebenenfalls auf den Maschinenzustand, rückschließen zu können.

Statt wie in Fig. 1 und 2 gezeigt eine "Normaldifferenzspule" zu verwenden, die zwei subtraktiv geschaltete Spulen umfaßt, könnte man auch eine Multidifferenzsspule verwenden, die dann beispielsweise vier Empfängerspulen aufweist, wobei jede der beiden Empfängerspulen 12 und 14 der Normaldifferenzspule durch zwei gegeneinander geschaltete Empfängerspulen ersetzt ist. Eine Multidifferenzspule hat eine bessere Rauschunterdrückung, d.h. ein besseres Signal-Rausch-Verhältnis, als eine Normaldifferenzspule, und die Signalform ist ausgeprägter. Allerdings ist der Aufbau aufwändiger und die Signalamplituden sind kleiner. Auch erhält man gegebenenfalls störende Vor- und Nachschwinger.

Im folgenden soll das von der Differenzspule beim Durchgang eines Partikels erzeugte Signal als "Differenzsignal" bezeichnet werden.

Die Größe der erfassten Partikel liegt beispielsweise zwischen 1 und 25 $\mu$m. Größere Partikel werden üblicherweise aus dem Schmiermittel ausgefiltert, um Schäden an der Maschine zu verhindern.

Zweckmäßigerweise wird die Zahl der erfassten Partikel pro Zeit ermittelt, woraus die Konzentration der elektrisch leitenden Partikel in der Flüssigkeit ermittelt werden kann, da der Flüssigkeitsdurchsatz üblicherweise bekannt und im wesentlichen konstant ist.

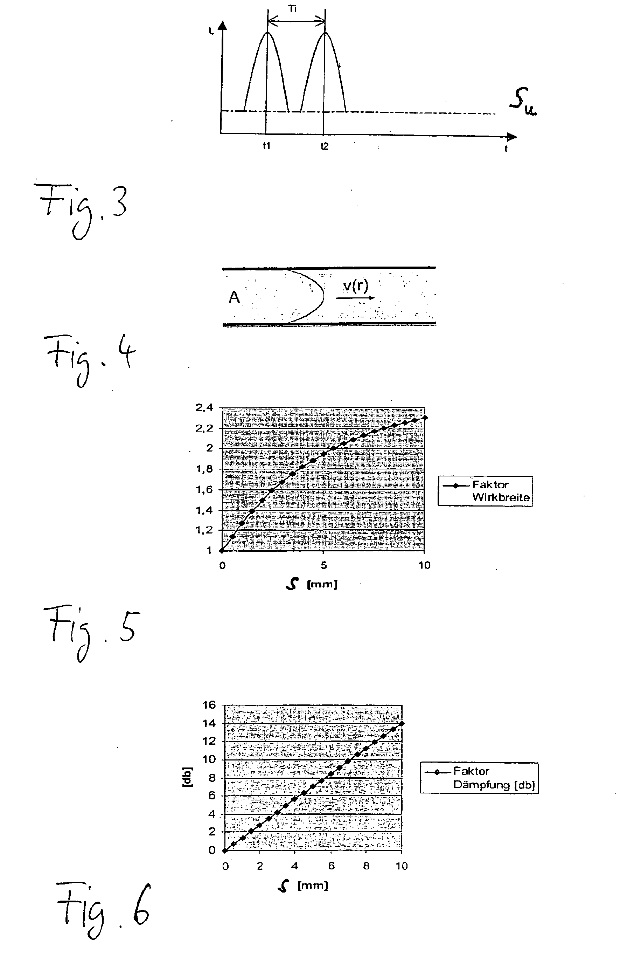

Aus der Auswertung des Differenzsignals der Empfängerspulen kann nicht nur der Durchgang eines Partikels erfasst werden, sondern es können auch zusätzliche Informationen gewonnen werden, insbesondere bezüglich der radialen Position des Partikels beim Durchgang durch die Empfängerspulen, d.h. dem radialen Abstand des Partikels von der Wand der Empfängerspulen, der Fließgeschwindigkeit des erfassten Partikels, der Größe des erfassten Partikels sowie des Volumenstroms, d.h. der über den Querschnitt des Rohrstücks 10 gemittelte Strömungsgeschwindigkeit der Flüssigkeit 16. Wie solche zusätzlichen Information gewonnen werden kann, soll im folgenden anhand der Figuren 3 bis 6 beispielhaft erläutert werden.

In der Regel besteht bei einem Partikelzähler auch der Wunsch, den Volumenstrom zu erfassen, um die gezählten Partikel auf ein Volumen normieren zu können (Partikel/*ml*) und bestehenden Normtabellen zuzuordnen. Dabei muss bei gegebenem Rohrdurchmesser die Geschwindigkeit der Flüssigkeit gemessen werden, woraus man dann das Volumen des Schmiermittels berechnen kann, das während der Messzeit (typischerweise 1 bis 30 Minuten) den Partikelzähler passiert hat. Während es hierzu grundsätzlich viele unterschiedliche - mehr oder weniger aufwändige - Lösungen, z.B. basierend auf Temperaturmessung, Ultraschall, Impulse aus mechanischen Mühlrädchen, etc., gibt, ist es besonders vorteilhaft, den Volumenstrom aus den für die Partikelzählung ohnehin erfassten Wirbelstromsignalen abzuleiten. Dadurch kann eine Lösung ohne zusätzliche Sensorik realisiert werden, was zu geringeren Kosten, geringerer Ausfallwahrscheinlichkeit und geringerem Platzbedarf führt. Zwar kann man in diesem Fall die Fließgeschwindigkeit nur dann erfassen, wenn Partikel erkannt werden. Dies ist jedoch in der Regel kein Problem, da ohnehin eine Messung nur stattfindet, wenn auch Partikel erkannt werden.

Ferner ist es bei einem Partikelzähler üblicherweise von Vorteil, die Größe der erfassten Partikel abzuschätzen und die erfassten Partikel nach der abgeschätzten Größe zu klassifizieren, um eine möglichst aussagekräftige Charakterisierung des Maschinenzustands zu erzielen. Beispielsweise kann bei über Überschreiten eines vorgegebenen Grenzwerts für die Zahl der insgesamt oder für eine bestimmte Größenklasse erfassten Partikel pro Zeit ein Alarmsignal ausgegeben werden.

Da sowohl die Amplitude als auch der zeitliche Verlauf des von einem Partikel verursachten Differenzsignals von der radialen Position des Partikels im Rohrstück 10 bzw. in der Spule abhängt, ist es sowohl für die Geschwindigkeitsmessung bzw. Volumenstrommessung als auch für die Partikelgrössenmessung vorteilhaft, die radiale Position des Partikels abzuschätzen und die Geschwindigkeitsmessung bzw. die Größenmessung entsprechend zu korrigieren.

In Fig. 3 ist ein Beispiel für den idealisierten Verlauf des Betrags des Differenzsignals einer Normaldifferenzspule, wie z.B. der von den Empfängerspulen 12, 14 von Fig. 1 gebildeten Differenzspule, gezeigt, wobei die Messwerte unterhalb eines Amplitudenschwellwerts $S_u$ abgeschnitten wurden (das abgeschnittene Signal umfasst Grundrauschen sowie Anfang und Ende des Differenzsignals). Solange die Signalamplitude oberhalb des Schwellwerts $S_u$ liegt, wird das Signal aufgezeichnet und im Prozessor der Auswerteeinheit gespeichert. Man erhält zwei getrennte Signalbögen, die jeweils ein Maximum beim Zeitpunkt $t_1$ bzw. $t_2$ aufweisen. Das jeweilige Amplitudenmaximum, d.h. der jeweilige Zeitpunkt $t_1$ bzw. $t_2$, kann beispielsweise durch parabolische Ausgleichsrechnung oder einfacher durch eine Maximalwertsuche bestimmt werden. Entscheidend für die nachfolgende Auswertung ist der Wert des Amplitudenmaximums $A_i$ sowie die Zeitdifferenz $T_i$, die sich aus der Differenz zwischen $t_1$ und $t_2$ ergibt. Für jedes Zählereignis, d.h. für jeden gefundenen Partikel i, wird die entsprechende Maximalamplitude $A_i$ des Differenzsignals (ggf. auch komplex) und die dazugehörige Zeitdifferenz $T_i$ abgespeichert.

Da die beiden Differenzspulen 12, 14 in axialer Richtung einen Abstand $d_{Sp}$ aufweisen, ist die Zeitdifferenz $T_i$ in erster Näherung proportional zur Strömungsgeschwindigkeit $v_i$ des Partikels. Der axiale Abstand der Spulen schlägt sich dabei in der sogenannten Wirkbreite der Differenzspule nieder. Grundsätzlich gilt der Zusammenhang $v_i = k*WB/T_i$. Der Faktor k hängt von bestimmten Eigenschaften der Differenzspule ab und kann im Werk einmalig für den jeweiligen Typ des Partikelzählers ermittelt werden.

Die Wirkbreite WB hängt von der radialen Position des Partikels beim Durchgang durch die Differenzspule ab, wobei sie mit zunehmendem radialem Abstand des Partikels von der Spulenwand zunimmt. Dieser Tatsache kann Rechnung getragen werden, indem man werksseitig für den jeweiligen Typ des Partikelzählers die Abhängigkeit der Wirkbreite von der radialen Position des Partikels empirisch ermittelt. In Fig. 5 ist ein Beispiel für die relative Vergrößerung der Wirkbreite mit zunehmendem radialen Partikelabstand s gezeigt. Anhand einer solchen empirischen Kurve kann, wie dies nachstehend genauer beschrieben wird, die gemessene Zeitdifferenz $T_i$ hinsichtlich des radialen Abstands des Partikels i korrigiert werden.

Grundsätzlich ist die Maximalamplitude $A_i$ des Differenzsignals ein Maß für die Größe des Partikels i. Jedoch ist hierbei zu berücksichtigen, dass die Dämpfung der Differenzspule von der radialen Position s des Partikels abhängt, so dass für eine verlässliche Abschätzung der Partikelgröße die gemessene Maximalamplitude $A_i$ entsprechend korrigiert werden muss. Dies kann dadurch erfolgen, dass die Abhängigkeit der Dämpfung des Differenzsignals vom radialen Abstand s von der Spulenwand für den jeweiligen Typ des Partikelzählers werksseitig empirisch ermittelt wird. In Fig. 6 ist ein Beispiel für eine solche Korrekturkurve gezeigt. Je weiter ein Partikel vom Spulensystem radial entfernt ist, um so schwächer wird die Signalamplitude. Zwar spielt auch hier die Größe des Partikels eine gewisse Rolle; die Dämpfungsfunktion bezüglich des Abstands wird aber im wesentlichen gleich verlaufen.

Ferner ist bezüglich der Teilchengeschwindigkeiten noch zu berücksichtigen, dass die Geschwindigkeit einer laminaren Strömung in einem Rohr in bekannter Weise vom radialen Abstand r von der Rohrwand abhängt, wobei die Abhängigkeit parabolisch ist und die maximale Strömungsgeschwindigkeit im Zentrum des Rohrs liegt. Man wird also aus diesem Grund eine bestimmte Verteilung der Partikelgeschwindigkeiten und somit der gemessenen Zeitdifferenzen $T_i$ erhalten.

Bei der Auswertung der Differenzsignale ist es zweckmäßig, folgende Annahmen zu machen:

1. Während eines Messintervalls von typischerweise 1 bis 30 Minuten bleibt der Volumenstrom im wesentlichen konstant, wobei dies natürlich auch von der jeweiligen Anlage abhängt. In der Praxis treten Geschwindigkeitsschwankungen im wesentlichen beim Anfahren der Anlage auf. In dieser Zeit wird aber ohnehin keine Partikelmessung durchgeführt. Ansonsten ergeben sich Änderungen des Volumenstroms hauptsächlich durch Schwankungen der Umgebungstemperatur (Einfluss auf die Viskosität des Schmiermittels) und durch die sich langsam verändernde Durchlässigkeit der Schmiermittelfilter. Nur bei Störfällen kann es zu schnellen Änderungen der Fließgeschwindigkeit kommen, wenn z.B. ein Filter bricht.

2. Der radiale Abstand s der Partikel zum Spulensystem ist statistisch gesehen gleich verteilt.

3. Es tritt keine turbulente Strömung auf. Dies kann durch Leitelemente sichergestellt werden.

4. Alle Partikel sind in ihrer Ausdehnung viel kleiner als die Wirkbreiten der Spulen.

Im folgenden wird ein Beispiel für die Auswertung der Differenzsignale beschrieben.

Wie bereits erwähnt, werden während eines Messintervalls von typischerweise 1 bis 30 Minuten die Maximalamplitude $A_i$ und die Zeitdifferenz $T_i$ für jeden erfassten Partikel i gespeichert. Um eine verlässliche Auswertung zu ermöglichen, sollte eine gewisse Mindestanzahl von Partikeln erfasst werden. Gegebenenfalls muss bei kleiner Partikelkonzentration das Messintervall entsprechend verlängert werden. Als Ergebnis erhält man eine gewisse Verteilung der Maximalamplituden und der Zeitdifferenzen. Die kleinsten Zeitdifferenzen $T_i$ repräsentieren dabei Partikel im Zentrum der Spulenanordnung, d.h. $s = r_0$. Für die Verteilung der Strömungsgeschwindigkeiten v(r) einer laminaren Strömung in einem Rohr gilt:

$$v(r) = \frac{\Delta p}{4 \cdot l \cdot \eta} \cdot (r_0{}^2 - r^2)$$

Dabei ist $\Delta p$ die Druckdifferenz in Pascal, $l$ ist die Länge des Rohrs in Meter und $\eta$ ist die kinematische Viskosität in Pa s.

Die tatsächliche Maximalgeschwindigkeit im Rohr kann abgeschätzt werden, indem man die kleinste gemessene Zeitdifferenz $T_i$ mit dem "Faktor Wirkbreite" für $r_0$ dividiert:

$$T' = Min\ (T_i)/FW(r_0).$$

Da wir den tatsächlichen geometrischen Spulenabstand $d_{Sp}$ und jetzt auch die korrigierte Zeit $T'$ und kennen, kann die Maximalgeschwindigkeit v$_{max}$ berechnet werden:

$$v_{max} = d_{Sp}/T'$$

Daraus ergibt sich für die mittlere Geschwindigkeit $V_{mean} = V_{max}/2$. Für den Volumenstrom gilt:

$$I = v_{mean}\ r_0{}^2\pi.$$

Da man nun $V_{max}$ kennt, kann die Konstante $\Delta p/\eta$ bestimmt werden:

$$c = \frac{\Delta p}{l \cdot \eta} = \frac{4 \cdot v_{max}}{r_o{}^2}$$

Somit ist nun die tatsächliche Geschwindigkeitsverteilung v(r) bzw. v(s) im Rohr bekannt. Damit kann man nun korrigierte Zeitdifferenzen $T'_i(s)$ entsprechend einer berechneten Wirkbreite bestimmen:

$$T'(s) = \frac{d_{Sp}}{v(s) \cdot FW(s)} = \frac{d_{Sp}}{\frac{c}{4}\left(r_o{}^2 - (r_o - s)^2\right) \cdot FW(s)}$$

Damit erhält man nun beispielsweise eine Tabelle mit den Werten $T'_i(s)$ in Abhängigkeit vom radialen Abstand $s$ des Partikels $i$ zur Spule. Im einfachsten Fall kann man diese Tabelle als Look-up-Table benutzen, um jedem gefundenen Partikel i einen radialen Abstand $s$ zur Spule zuzuordnen. Dabei nimmt man den gemessenen Wert $T_i$ und sucht nach dem nächstgelegenen Wert $T'_i(s)$ in der Tabelle.

Mittels einer solchen Abstandszuordnung kann man nicht nur die gemessene Zeitdifferenz und damit die berechnete Partikelgeschwindigkeit korrigieren, sondern man kann auch die jeweils gemessene Maximalamplitude $A_i$ anhand der vorab empirisch ermittelten Abhängigkeit der Differenzsignaldämfung vom radialen Partikelabstand $s$ korrigieren. Dabei wird der Amplitudenwert im einfachsten Fall auf einen Skalar reduziert, zweckmäßigerweise auf den Maximalwert der Betragsdarstellung des Differenzsignals von Fig. 3. Dieser Wert wird dann mit dem dazugehörigen Dämpfungswert korrigiert. Anschließend wird der Wert anhand der Bewertungsschwellen (z.B. acht Bewertungsschwellen) bewertet. Jeder Bewertungsbereich entspricht dabei einem Größenbereich der Partikel. Für jeden Bereich gibt es einen Zähler, der inkrementiert wird, wenn die gemessene Partikelamplitude in diesen Bereich fällt. Nach dem Messintervall wird das Gesamtvolumen der Flüssigkeit anhand der Messdauer und dem ermittelten Volumenstrom berechnet und entsprechend der Zählerstände eine Verunreinigungsklasse ausgewiesen, z.B. nach ISO 4406. Alternativ kann die Amplitudenauswertung anhand eines dem jeweiligen Partikel anhand des Differenzsignals zugeordneten Vektors erfolgen (dabei wird dann nicht nur der maximale Amplitudenwert sondern auch die Phase berücksichtigt).

Es versteht sich, dass die empirisch ermittelten Korrekturfunktionen gemäß Fig. 5 und Fig. 6 mittels Ausgleichrechnung durch geeignete Funktionen, z.B. genäherte Parabeln sowie deren Umkehrfunktion, dargestellt werden können. In diesem Fall kann der Look-up-Table entfallen.

Unmittelbar nach dem Einschalten des Partikelzählers ist die Strömungsgeschwindigkeit noch nicht bekannt ist und somit können Partikel dann u.U. nicht zuverlässig diskretisiert werden, da die Zuordnung der einzelnen Betragssignalbögen zum Differenzsignal ohne Kenntnis des zu erwartenden Bereichs der Zeitdifferenzen $T_i$ zu einem bestimmten Ereignis, nämlich dem Durchgang eines Partikels, nicht immer zuverlässig möglich ist. Um diese Probleme zu umgehen, kann als "Anfahrhilfe" ein Signal nach dem Überschreiten des unteren Schwellwerts $S_u$ in einer Länge aufgezeichnet werden, die für die Erkennung einer Mindestströmungsgeschwindigkeit ausreichend ist. Die erfaßten Differenzsignale können dann anhand eines solchermaßen erfaßten typischen Verlaufs separiert bzw. einzelnen Partikeldurchgängen zugeordnet werden. Eine solche Separation kann beispielsweise mittels Kreuzkorrelation mit Variation der vorgegebenen Wirkbreiten bzw. Zeitdifferenzen $T_i$ erfolgen. Dabei wird die vorgegebene Wirkbreite bzw. die vorgegebene Zeitdifferenz so variiert, dass die Amplituden der Kreuzkorrelationsfunktion maximiert werden.

## Patentansprüche

1. Verfahren zum Erfassen von elektrisch leitenden Partikeln (20) in einer in einem Rohrstück (10) strömenden Flüssigkeit (16), wobei mittels einer das Rohrstück (10) umgebenden Senderspule (18) Wirbelströme in den Partikeln (20) induziert werden, wobei mittels mindestens einer ersten das Rohrstück (10) umgebenden induktiven Empfängerspule (12) und einer zweiten das Rohrstück (10) umgebenden, axial zur ersten Empfängerspule (12) beabstandeten, induktiven Empfängerspule (14), die im Bereich der Senderspule (18) angeordnet sind und subtraktiv geschaltet sind, ein Differenzsignal entsprechend den von der Senderspule (18) induzierten Wirbelströmen ausgegeben wird, wobei die Senderspule (18) die Primärseite und die Empfängerspulen (12, 14) die Sekundärseite einer transformatorischen Anordnung bilden, und wobei das Differenzsignal ausgewertet wird, um den Durchgang von elektrisch leitenden Partikeln (20) in dem Rohrstück (10) zu erfassen,
**dadurch gekennzeichnet, dass**
der radiale Abstand der Partikel zur Spule in Abhängigkeit von der Zeitdifferenz des Partikeldurchgangs durch die Empfängerspulen ermittelt wird,
wobei der abgeschätzte radiale Abstand der erfassten Partikel (20) von der Empfängerspulenwand bei der Abschätzung der Strömungsgeschwindigkeit der Partikel verwendet wird,
oder wobei der abgeschätzte radiale Abstand jedes erfassten Partikel (20) von der Empfängerspulenwand bei der Abschätzung der Größe der Partikel verwendet wird.

2. Verfahren gemäß Anspruch 1, **dadurch gekennzeichnet, dass** das Rohrstück (10) Teil eines Schmiermittel-Kreislaufs ist.

3. Verfahren gemäß Anspruch 1, **dadurch gekennzeichnet, dass** es sich bei der Flüssigkeit (16) um Schmieröl einer Maschine handelt.

4. Verfahren gemäß einem der Ansprüche 1 bis 3, **dadurch gekennzeichnet, dass** die Sendefrequenz der Senderspule (18) zwischen 20 kHz und 500 kHz liegt.

5. Verfahren gemäß einem der Ansprüche 1 bis 4, **dadurch gekennzeichnet, dass** die Größe der erfassten Partikel (20) zwischen 1 und 25 $\mu$m liegt.

6. Verfahren gemäß einem der Ansprüche 1 bis 5, **dadurch gekennzeichnet, dass** die Zahl der erfassten Partikel (20) pro Zeit ermittelt wird.

7. Verfahren gemäß einem der Ansprüche 1 bis 6, **dadurch gekennzeichnet, dass** die Konzentration der elektrisch leitenden Partikel (20) in der Flüssigkeit (16) ermittelt wird.

8. Verfahren gemäß einem der Ansprüche 1 bis 7, **dadurch gekennzeichnet, dass** bei Überschreiten eines vorgegebenen Grenzwerts für die Zahl der erfassten Partikel (20) pro Zeit ein Alarmsignal ausgegeben wird.

9. Verfahren gemäß einem der Ansprüche 1 bis 8, **dadurch gekennzeichnet, dass** nur Differenzsignale bei der Partikelerfassung verwendet werden, deren Amplitude einen bestimmten Schwellwert überschreitet.

10. Verfahren gemäß Anspruch 9, **dadurch gekennzeichnet, dass** das Differenzsignal aufgezeichnet wird, solange die Signalamplitude den Schwellwert überschreitet.

11. Verfahren gemäß einem der Ansprüche 1 bis 10, **dadurch gekennzeichnet, dass** die Strömungsgeschwindigkeit der erfassten Partikel (20) aus dem Differenzsignal abgeschätzt wird.

12. Verfahren gemäß Anspruch 11, **dadurch gekennzeichnet, dass** die Strömungsgeschwindigkeit der erfassten Partikel (20) aus dem zeitlichen Abstand der Extremwerte des Differenzsignals abgeschätzt wird.

13. Verfahren gemäß Anspruch 12, **dadurch gekennzeichnet, dass** die Strömungsgeschwindigkeit der erfassten Partikel (20) aus dem zeitlichen Abstand der Maxima des Betrags des Differenzsignals abgeschätzt wird.

14. Verfahren gemäß Anspruch 12 oder 13, **dadurch gekennzeichnet, dass** die Verteilung der ermittelten zeitlichen Abstände bestimmt wird und einer statistischen Analyse unterzogen wird.

15. Verfahren gemäß Anspruch 14, **dadurch gekennzeichnet, dass** bei der statistischen Analyse der ermittelten zeitlichen Abstände die theoretische radiale Verteilung der Strömungsgeschwindigkeit in einer laminaren Strömung berücksichtigt wird.

16. Verfahren gemäß Anspruch 15, **dadurch gekennzeichnet, dass** aus dem Maximum der abgeschätzten Partikelgeschwindigkeiten unter Berücksichtigung des geometrischen Abstands der Empfängerspulen die Strömungsgeschwindigkeit der Flüssigkeit (16) ermittelt wird.

17. Verfahren gemäß Anspruch 17, **dadurch gekennzeichnet, dass** vorab die Abhängigkeit der Wirkbreite der Empfängerspulen (12, 14) vom radialen Abstand der erfasstem Partikels (20) von der Empfängerspulenwand empirisch ermittelt wird.

18. Verfahren gemäß Anspruch 18, **dadurch gekennzeichnet, dass** bei der Abschätzung des radialen Abstands der erfassten Partikel (20) von der Empfängerspulenwand die theoretische radiale Verteilung der Strömungsgeschwindigkeit in einer laminaren Strömung, der geometrische Abstand der Empfängerspulen (12, 14) sowie die vorab ermittelte Abhängigkeit der Wirkbreite der Empfängerspulen vom radialen Abstand der Partikel berücksichtigt werden.

19. Verfahren gemäß einem der Ansprüche 1 bis 19, **dadurch gekennzeichnet, dass** die Größe der erfassten Partikel (20) aus dem Differenzsignal abgeschätzt wird und die erfassten Partikel nach der abgeschätzten Größe klassifiziert werden.

20. Verfahren gemäß Anspruch 21, **dadurch gekennzeichnet, dass** vorab die Abhängigkeit der Amplitude des Differenzsignals vom radialen Abstand des Partikels (20) von der Empfängerspulenwand empirisch ermittelt wird.

21. Verfahren gemäß Anspruch 22, **dadurch gekennzeichnet, dass** bei der Abschätzung der Größe der erfassten Partikel (20) die theoretische radiale Verteilung der Strömungsgeschwindigkeit in einer laminaren Strömung, der geometrische Abstand der Empfängerspulen (12, 14), die vorab ermittelte Abhängigkeit der Wirkbreite der Empfängerspulen vom radialen Abstand der Partikel von der Empfängerspulenwand sowie die vorab ermittelte Abhängigkeit der Amplitude des Differenzsignals vom radialen Abstand der Partikel von der Empfängerspulenwand berücksichtigt werden.

**Claims**

1. Method for detecting electrically conductive particles (20) in a liquid (16) flowing in a pipe section (10), wherein eddy currents are induced in the particles (20) using a transmitter coil (18) surrounding the pipe section (10), wherein a difference signal corresponding to the eddy currents induced by the transmitter coil (18) is output using at least one first inductive receiver coil (12) surrounding the pipe section (10) and using a second inductive receiver coil (14) surrounding the pipe section (10) and at an axial spacing from the first receiver coil (12), which receiver coils are arranged in the region of the transmitter coil (18) and are connected subtractively, wherein the transmitter coil (18) forms the primary side and the receiver coils (12, 14) form the secondary side of a transformer-based arrangement and wherein the difference signal is evaluated in order to detect the passage of electrically conductive particles (20) in the pipe section (10),

**characterized in that**

the radial spacing of the particles from the coil is ascertained in dependence on the time difference of the particle passage through the receiver coils, wherein the estimated radial spacing of the detected particles (20) from the receiver coil wall is used when estimating the flow speed of the particles,

or wherein the estimated radial spacing of each detected particle (20) from the receiver coil wall is used when estimating the size of the particles.

2. Method according to Claim 1, **characterized in that** the pipe section (10) is part of a lubrication circuit.

3. Method according to Claim 1, **characterized in that** the liquid (16) is a lubricant oil of a machine.

4. Method according to one of Claims 1 to 3, **characterized in that** the transmission frequency of the transmitter coil (18) is between 20 kHz and 500 kHz.

5. Method according to one of Claims 1 to 4, **characterized in that** the size of the detected particles (20) is between 1 and 25 $\mu$m.

6. Method according to one of Claims 1 to 5, **characterized in that** the number of the detected particles (20) is ascertained per unit time.

7. Method according to one of Claims 1 to 6, **characterized in that** the concentration of the electrically conductive particles (20) in the liquid (16) is ascertained.

8. Method according to one of Claims 1 to 7, **characterized in that** an alarm signal is output when a predetermined limit value for the number of detected particles (20) per unit time is exceeded.

9. Method according to one of Claims 1 to 8, **characterized in that** only difference signals whose amplitude exceeds a specific threshold value are used in the particle detection.

10. Method according to Claim 9, **characterized in that** the difference signal is recorded for as long as the signal amplitude exceeds the threshold value.

11. Method according to one of Claims 1 to 10, **characterized in that** the flow speed of the detected particles (20) is estimated from the difference signal.

12. Method according to Claim 11, **characterized in that** the flow speed of the detected particles (20) is estimated from the time interval between the extreme values of the difference signal.

13. Method according to Claim 12, **characterized in that** the flow speed of the detected particles (20) is estimated from the time interval between the maxima of the magnitude of the difference signal.

14. Method according to Claim 12 or 13, **characterized in that** the distribution of the ascertained time intervals is determined and subjected to a statistical analysis.

15. Method according to Claim 14, **characterized in that** the theoretical radial distribution of the flow speed in a laminar flow is taken into account in the statistical analysis of the ascertained time intervals.

16. Method according to Claim 15, **characterized in that** the flow speed of the liquid (16) is ascertained from the maximum of the estimated particle speeds, taking into account the geometric spacing of the receiver coils.

17. Method according to Claim 1, **characterized in that** the dependence of the effective width of the receiver coils (12, 14) on the radial spacing of the detected particles (20) from the receiver coil wall is empirically ascertained in advance.

18. Method according to Claim 17, **characterized in that** the theoretical radial distribution of the flow speed in a laminar flow, the geometric spacing of the receiver coils (12, 14) and the dependence of the effective width of the receiver coils, ascertained in advance, on the radial spacing of the particles are taken into account in the estimation of the radial spacing of the detected particles (20) from the receiver coil wall.

19. Method according to one of Claims 1 to 18, **characterized in that** the size of the detected particles (20) is estimated from the difference signal and the detected particles are classified according to the estimated size.

20. Method according to Claim 19, **characterized in that** the dependence of the amplitude of the difference signal on the radial spacing of the particle (20) from the receiver coil wall is empirically ascertained in advance.

21. Method according to Claim 20, **characterized in that** the theoretical radial distribution of the flow speed in a laminar flow, the geometric spacing of the receiver coils (12, 14), the dependence of the effective width of the receiver coils, ascertained in advance, on the radial spacing of the particles from the receiver coil wall and the dependence of the amplitude of the difference signal, ascertained in advance, on the radial spacing of the particles from the receiver coil wall are taken into account in the estimation of the size of the detected particles (20).


**Revendications**

1. Procédé de détection de particules électriquement conductrices (20) dans un liquide (16) s'écoulant dans un élément tubulaire (10), dans lequel des courants de Foucault sont induits dans les particules (20) au moyen d'une bobine d'émission (18) entourant l'élément tubulaire (10), dans lequel un signal de différence correspondant aux courants de Foucault induit par la bobine d'émission (18) sont délivrés au moyen d'au moins une première bobine de réception inductive (12) entourant l'élément tubulaire (10) et d'une seconde bobine de réception inductive (14) espacée axialement de la première bobine de réception (12) et entourant l'élément tubulaire (10), lesquelles bobines sont disposées dans la région de la bobine d'émission (18) et sont connectées de manière soustractive, dans lequel la bobine d'émission (18) forme le côté primaire et les bobines de réception (12, 14) forment le côté secondaire d'un dispositif transformateur, et dans lequel le signal de différence est traité pour détecter le passage de particules électriquement conductrices (20) dans l'élément tubulaire (10),
   **caractérisé en ce que**
   la distance radiale des particules par rapport à la bobine est déterminée en fonction de la différence de temps entre les passages des particules à travers les bobines de réception, dans lequel la distance radiale estimée des particules détectées (20) par rapport à la paroi des bobines de réception est utilisée lors de l'estimation de la vitesse d'écoulement des particules, ou dans lequel la distance radiale estimée de chaque particule détectée (20) par rapport à la paroi des bobines de réception est utilisée lors de l'estimation de la taille des particules.

2. Procédé selon la revendication 1, **caractérisé en ce que** l'élément tubulaire (10) fait partie d'un circuit de lubrifiant.

3. Procédé selon la revendication 1, **caractérisé en ce que** le liquide (16) est une l'huile de lubrification d'une machine.

4. Procédé selon l'une quelconque des revendications 1 à 3, **caractérisé en ce que** la fréquence d'émission de la bobine d'émission (18) est comprise entre 20 kHz et 500 kHz.

5. Procédé selon l'une quelconque des revendications 1 à 4, **caractérisé en ce que** la taille des particules détectées (20) est comprise entre 1 et 25 $\mu$m.

6. Procédé selon l'une quelconque des revendications 1 à 5, **caractérisé en ce qu'**on détermine le nombre des particules détectées (20) par unité de temps.

7. Procédé selon l'une quelconque des revendications 1 à 6, **caractérisé en ce qu'**on détermine la concentration des particules électriquement conductrices (20) dans le liquide (16).

8. Procédé selon l'une quelconque des revendications 1 à 7, **caractérisé en ce qu'**un signal d'alarme est émis lors d'un dépassement d'une valeur limite prédéterminée du nombre des particules détectées (20) par unité de temps.

9. Procédé selon l'une quelconque des revendications 1 à 8, **caractérisé en ce que**, lors de la détection des particules, on n'utilise que des signaux de différence dont l'amplitude dépasse une valeur de seuil déterminée.

**10.** Procédé selon la revendication 9, **caractérisé en ce qu'**on enregistre le signal de différence tant que l'amplitude du signal dépasse la valeur de seuil.

**11.** Procédé selon l'une quelconque des revendications 1 à 10, **caractérisé en ce que** la vitesse d'écoulement des particules détectées (20) est estimée à partir du signal de différence.

**12.** Procédé selon la revendication 11, **caractérisé en ce que** la vitesse d'écoulement des particules détectées (20) est estimée à partir de l'intervalle de temps entre les valeurs extrêmes du signal de différence.

**13.** Procédé selon la revendication 12, **caractérisé en ce que** la vitesse d'écoulement des particules détectées (20) est estimée à partir de l'intervalle de temps entre les maxima de l'amplitude du signal de différence.

**14.** Procédé selon la revendication 12 ou 13, **caractérisé en ce que** la distribution des intervalles de temps obtenus est déterminée et **en ce qu'**elle est soumise à une analyse statistique.

**15.** Procédé selon la revendication 14, **caractérisé en ce que**, lors de l'analyse statistique des intervalles de temps obtenus, la distribution radiale théorique de la vitesse dans un écoulement laminaire est prise en compte.

**16.** Procédé selon la revendication 15, **caractérisé en ce que** la vitesse d'écoulement du liquide (16) est obtenue à partir du maximum des vitesses de particules estimées en tenant compte de la distance géométrique entre les bobines de réception.

**17.** Procédé selon la revendication 1, **caractérisé en ce que** la dépendance de la largeur efficace des bobines de réception (12, 14) vis-à-vis de la distance radiale des particules détectées (20) par rapport à la paroi des bobines de réception est déterminée empiriquement à l'avance.

**18.** Procédé selon la revendication 17, **caractérisé en ce que**, lors de l'estimation des distances radiales des particules détectées (20) par rapport à la paroi des bobines de réception, la distribution radiale de la vitesse d'écoulement dans un écoulement laminaire, la distance géométrique des bobines de réception (12, 14) ainsi que la dépendance déterminée à l'avance de la largeur efficace des bobines de réception vis-à-vis de la distance radiale des particules sont prises en compte.

**19.** Procédé selon l'une quelconque des revendications 1 à 18, **caractérisé en ce que** la taille des particules détectées (20) est estimée à partir du signal de différence et **en ce que** les particules détectées sont classées en fonction de la taille estimée.

**20.** Procédé selon la revendication 19, **caractérisé en ce que** la dépendance de l'amplitude du signal de différence vis-à-vis de la distance radiale de la particule (20) par rapport à la paroi de la bobine de réception est obtenue empiriquement à l'avance.

**21.** Procédé selon la revendication 20, **caractérisé en ce que**, lors de l'estimation la taille des particules détectées (20), la distribution radiale théorique de la vitesse d'écoulement dans un écoulement laminaire, la distance géométrique des bobines de réception (12, 14), la dépendance déterminée à l'avance de la largeur efficace des bobines de réception vis-à-vis de la distance radiale des particules par rapport à la paroi des bobines de réception et la dépendance déterminée à l'avance de l'amplitude du signal de différence vis-à-vis de la distance radiale des particules par rapport à la paroi des bobines de réception, sont prises en compte.

Fig. 1

Fig. 2

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2108717 A1
- DE 2840358 A1
- WO 2004081608 A
- WO 2004104561 A
- EP 0778937 A2
- EP 0451209 B1
- DE 3931497 A1
- DE 3117319 A1
- DE 4014756 A1
- US 3575050 A
- DE 2850246 A1
- EP 1979733 A1
- US 5001424 A1
- EP 1189058 A2